# EUROPEAN PATENT SPECIFICATION

(11) **EP 0 288 306 B1**
(45) Date of publication and mention of the grant of the patent: **13.12.1995**
(21) Application number: 88303657.6
(22) Date of filing: 22.04.1988
(51) Int. Cl.: C12N 15/31, C12P 21/02, C12Q 1/68, A61K 39/04

(54) **Diagnostics and vaccines for mycobacteria in public health, medical and veterinary practice**
Diagnostika und Impfstoffe für Mycobacteria in der Volksgesundheit, in der medizinischen und veterinären Praxis
Diagnostics et vaccins pour mycobactéria dans la santé publique, la pratique médicale et vétérinaire

(30) Priority: 24.04.1987 GB 8709803
(43) Date of publication of application: 26.10.1988
(73) Proprietor: BIOSCIENCE INTERNATIONAL, INC., Boston Massachusetts 02108 (US)
(72) Inventor: Hermon-Taylor, John Dept. of Surgery, Cranmer Terrace London SW 17 0RE (GB); McFadden, John-Jo Dept. of Surgery, Cranmer Terrace London SW17 0RE (GB)
(74) Representative: Goldin, Douglas Michael

(56) References cited:
- EP-A- 0 076 123
- EP-A- 0 079 139
- EP-A- 0 168 933
- EP-A- 0 173 339
- EP-A- 0 196 215
- EP-A- 0 229 442
- WO-A-84/02721
- WO-A-86/00019
- WO-A-88/00009
- WO-A-88/00977
- CHEMICAL ABSTRACTS, vol. 105, 1986, page 408, no. 222283v, Columbus, Ohio, US; J.T. CRAWFORD et al.: "Analysis of plasmids in Mycobacteriumaviumintracellulare isolates from persons with acquired immunodeficiencysyndrome"
- CHEMICAL ABSTRACTS, vol. 104, 1986, page 196, no. 63334y, Columbus, Ohio, US; A. LABIDI et al.
- CHEMICAL ABSTRACTS, vol. 104, 1986, page 156, no. 1677d, Columbus, Ohio, US; A.LABIDI et al.: "Cloning and expression of mycobacterial plasmid DNA in Eschberichia coli"
- CHEMICAL ABSTRACTS, vol. 96, 1982, page 296, no. 65372x, Columbus, Ohio, US; J.T. CRAWFORD et al.: "Characterization of plasmids from strains of Mycobacteriumavium-intracellulare"
- CHEMICAL ABSTRACTS, vol. 103, 1985, page 175, no. 117200b, Columbus, Ohio, US; P.T. HAMILTON et al.: "Structure of genes and insertion element in the methane-producing archaebacterium Methanobrevibacter smithii" & MOL.GEN.GENET. 1985, 200(1), 47-59

## Description

### INTRODUCTION.

It is estimated that 3 to 6 million people in the world die each year from leprosy and tuberculosis alone. Human disease due to atypical mycobacteria is increasing. Tuberculosis and paratuberculosis are common in livestock, account for major economic losses, and form a reservoir of infection for human disease. Despite BCG vaccination and some effective drugs, mycobacterial disease remains a major global problem.

Present methods for the identification and characterisation of mycobacteria in samples from human and animal diseases are by Zeil-Neilson staining, in-vitro and in vivo culture, biochemical testing and serological typing [1]. These methods are generally slow and do not readily discriminate between closely related mycobacterial strains and species particularly, for example, Mycobacterium paratuberculosis and Mycobacterium avium. Mycobacteria are widespread in the environment, and rapid methods do not exist for the identification of specific pathogenic strains from amongst the many environmental strains which are generally non-pathogenic. Difficulties with existing methods of mycobacterial identification and characterisation have increased relevance for the analysis of microbial isolates from Crohn's disease (Regional Ileitis) in humans [2] and Johne's disease in animals [3] (particularly cattle, sheep and goats) as well as for M. avium strains from AIDS patients with mycobacterial superinfections [4]. Although recognition of the causative agents of human leprosy and tuberculosis are clear, clinico-pathological forms of each disease exist, such as the tuberculoid form of leprosy, in which mycobacterial tissue abundance is low and identification correspondingly difficult. Improvements in the specific recognition and characterisation of mycobacteria may also increase in relevance if current evidence linking diseases such as rheumatoid arthritis to mycobacterial antigens is substantiated [5]. Emerging drugs resistance to mycobacteria including M. avium isolates from AIDS patients, and Mycobacterium tuberculosis from TB patients is an increasing problem. Rapid methods for the identification of mycobacteria in environmental and disease samples, for the specific recognition of pathogenic strains, for precisely distinguishing closely related mycobacterial strains, and for defining resistance patterns to antimycobacterial agents are badly needed.

### THE INVENTION

### 1. DNA probes for the specific identification of mycobacteria

A method of using DNA probes for the precise identification of mycobacteria and discrimination between closely related mycobacterial strains and species by genotype characterisation. The method of genotypic analysis is further applicable to the rapid identification of phenotypic properties such as drug resistance and pathogenicity.

DNA probes of the invention are derived from recombinant DNA libraries or synthesised using DNA sequence information from recombinant DNA clones derived from different mycobacterial strains and species. DNA clones are selected for their ability specifically to identify mycobacterial DNA from other microbial DNA. DNA probes of the invention are also selected for their ability to distinguish and differentiate between closely related mycobacterial strains and species such as Mycobacterium paratuberculosis and Mycobacterium avium, and different strains of mycobacteria including those isolated from AIDS patients. DNA probes of the method of the invention are also selected for their ability to identify and predict mycobacterial phenotypes, including drug resistance, by genotype characterisation.

### 2. Diagnostics and vaccines for public health, medical and veterinary use based upon Mycobacterial Insertion Sequences (ISM).

We have identified, for the first time, a family of related DNA insertion sequences (ISM) in the genome of disease isolates of mycobacteria. The first example of ISM (ISM-1) we identified and characterised in the clone pMB22 (Deposited with the National Collections of Industrial and Marine Bacteria, NCIB number 12461, Aberdeen, Scotland) from a genomic DNA library derived from a mycobacterial isolate from Crohn's disease (CD). We also identify ISM-1 in Mycobacterium paratuberculosis cultured from cattle and other animals suffering from Johne's disease (JD). We identify related sequences of the ISM family in mycobacterial disease isolates superinfecting humans with AIDS (acquired immune deficiency syndrome), atypical mycobacterial pneumonias in immunocompetent humans, and other mycobacterial disease isolates from animals and birds. We also identify ISM in isolates of Mycobacterium leprae and Mycobacterium lepraemurium . ISM sequences appear to be prevalent in pathogenic mycobacteria, but have not been identified in saprophytic mycobacteria.

All or part of the DNA sequence of each member of the ISM family may be used with or without enzymic amplification by polymerase chain reaction (PCR) or other techniques, as DNA or RNA probes in diagnostics for the specific recognition of pathogenic mycobacteria in environmental and disease samples and in in-vitro mycobacterial cultures. All or part of the proteins or peptides encoded, mutated or modified in expression by ISM may be used in immunodiagnostics or other diagnostics for a similar purpose whether based on recombinant proteins or synthetic peptides. Such ISM derived proteins or peptides may also be used as specific vaccines for the prevention of mycobacterial disease in medical and veterinary practise. Alteration of microbial phenotype by deletion or transfection of ISM in whole or in part may be used to engineer cultivateable microbial strains suitable for use as vaccines, in immunotherapy or diagnostics.

### SUMMARY OF THE FIGURES:

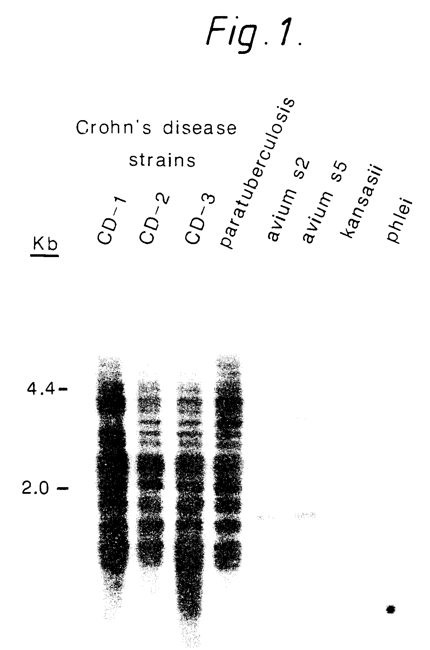

Figure 1 shows the multiple banding pattern or DNA "fingerprint" obtained when restricted genomic DNA from each of three separate Crohn's disease (CD) mycobacterial isolates and one Johne's disease (JD) M. paratuberculosis isolate are probed with pMB22 containing ISM-1 of the invention. Figure 1 also shows a different and much simpler banding pattern with restricted genomic DNA from 4 other species of mycobacteria which do not contain ISM-1 or homologous insertion sequences conferring pathogenicity. This figure illustrates how a shared and complex banding pattern can demonstrate identity between mycobacteria as between CD and JD isolates, as well as distinguish precisely between different mycobacteria.

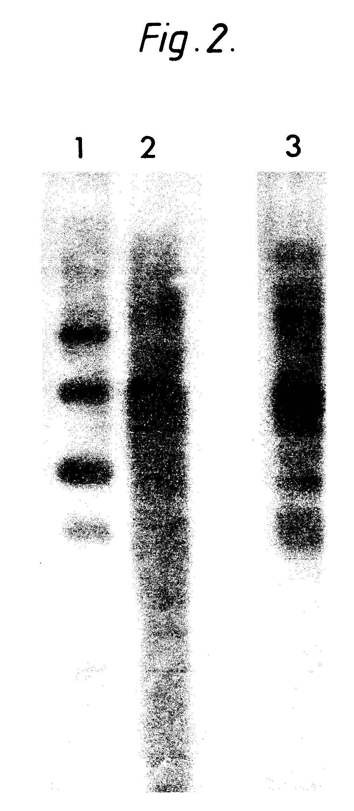

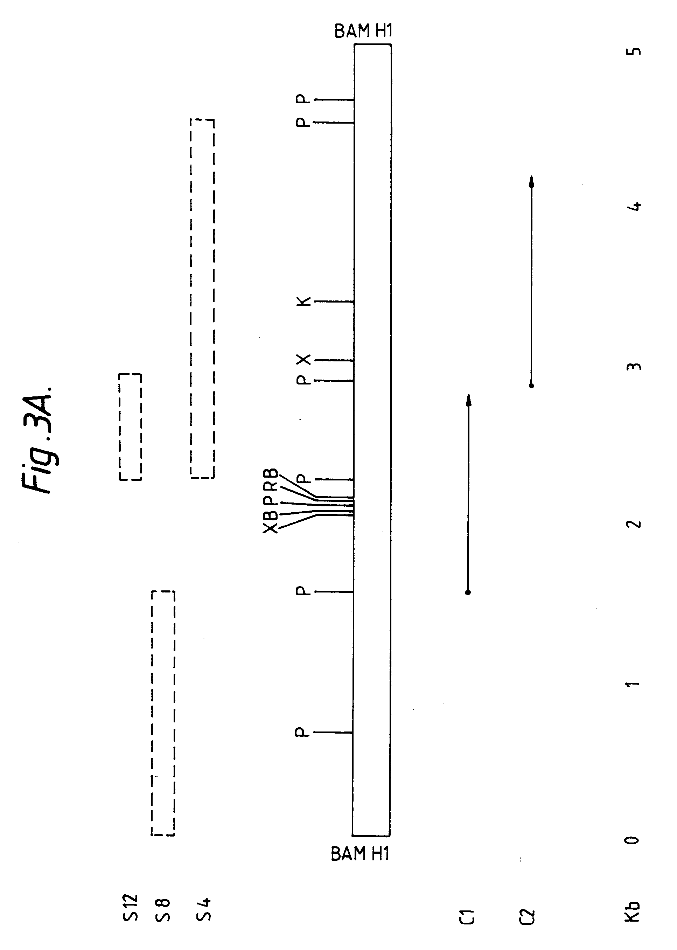

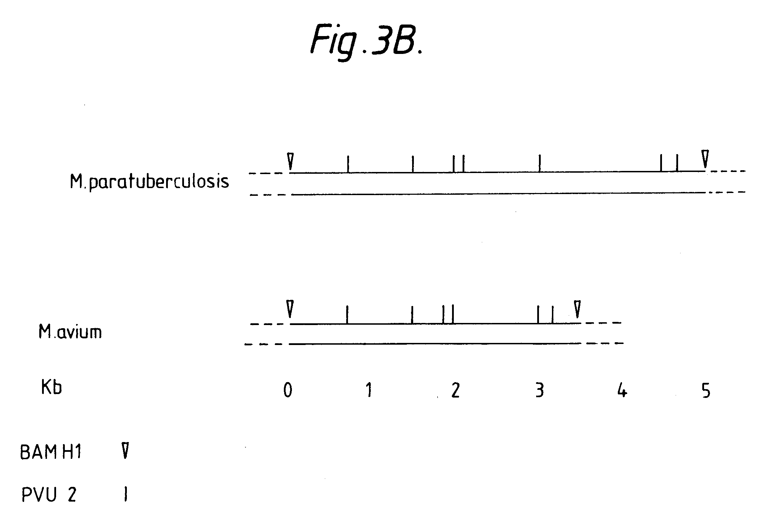

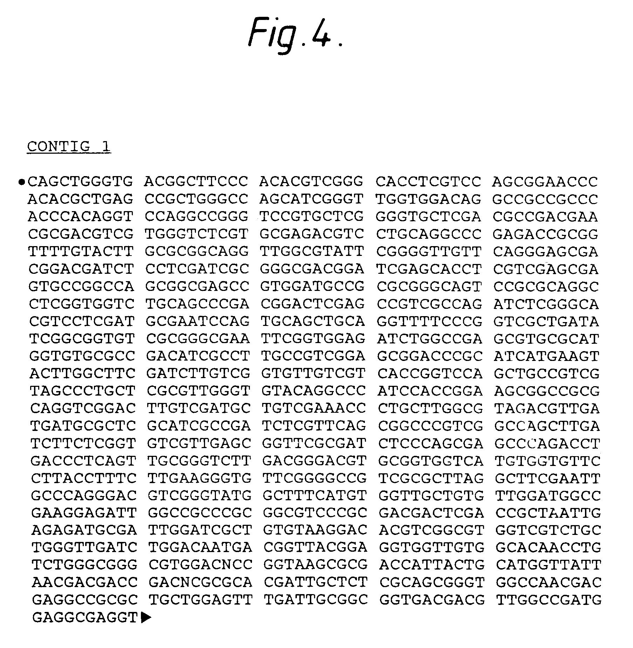

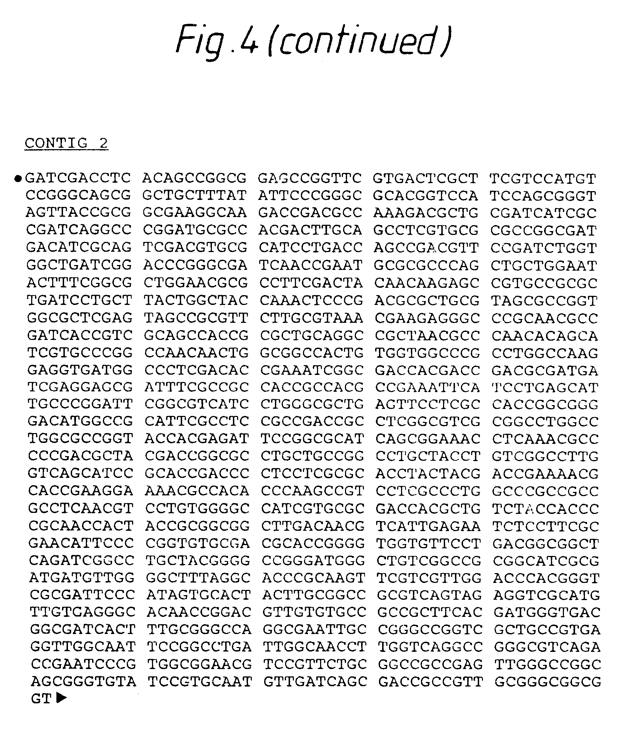

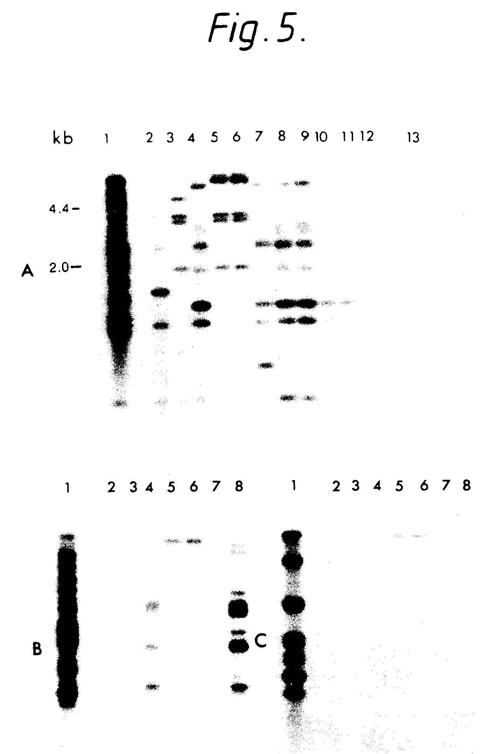

Figure 2 supports Figure 1 by showing identity between further independent CD mycobacterial isolates from Amsterdam, Holland and Los Angeles, U.S.A. both being different from M. avium. Figure 3 shows a restriction map which characterises the 5 kb of insert DNA containing the sequence ISM-1 of the invention, in the clone pMB22. Fig 3 also shows mapping data obtained from M. avium and M. paratuberculosis DNA indicating that DNA flanking ISM-1 in M. paratuberculosis is identical to DNA found at the same site in M. avium but without of course, an associated ISM-1. This indicates that ISM-1 is inserted into the genome of M. paratuberculosis at this site and is a likely cause of the divergent phenotype. Figure 4 further characterises the insert cloned in pMB22 with partial DNA sequence data spanning the ISM-1 insertion element. Figure 5 shows the multiple banding patterns obtained from a series of pathogenic strains of M. avium which are shown to contain other ISM sequences related to the insertion sequence ISM-1 found in pMB22. These related ISM sequences may be involved in pathogenicity of these and other mycobacteria, particularly those of the M. avium complex. Figure 5 also demonstrates the use of DNA fingerprinting to identify further pathogenic mycobacteria and distinguish them from environmental mycobacteria.

The ISM-1 insertion element is referred to in published literature (Vary *et al*, J. Clinical Microbiology 1990, 28; 933-937) as IS900. The present invention thus provides the plasmid pMB22 (NCIMB 12461) containing IS900 of mycobacterial origin, and a host cell transformed by said plasmid.

The invention further provides isolated DNA consisting essentially of a mycobacterial DNA sequence having at least 70% sequence homology with any contiguous stretch of at least 20 nucleotides of the IS900 insertion sequence carried by pMB22 (NCIMB 12461). Preferably, the homology is to the entire IS900 insertion sequence carried by pMB22 (NCIMB 12461).

The invention also provides DNA or RNA probes based on sequences of the invention optionally carrying a revealing label.

In another aspect, the invention provides a method of obtaining a mycobacterial Insertion Element or fragment thereof which comprises probing the DNA of a pathogenic mycobacteria with a probe according to the invention to identify an insertion sequence element or fragment thereof which segregates with mycobacterial pathogenicity, and isolating said insertion sequence element or fragment thereof.

Such insertion sequence elements or fragments thereof so obtained may be used in a method of detecting the presence or absence or a mycobacterial strain or phenotype. The method may comprise assaying a sample of mycobacterial DNA or RNA with a DNA or RNA according to the invention.

Such a method may be used for the precise identification and differentiation of closely related mycobacterial strains and species, including *M. paratuberculosis, M. avium-intracellulare* or *M. avium* complex or *M. leprae.*

The method may also be used wherein the or one of the mycobacteria cause AIDS AIDS superinfections or atypical tuberculosis, or has a phenotype which is pathogenic or causes drug resistance.

Particular applications of the method are wherein the mycobacterial strain causes Johne's disease in animals or Crohn's disease in humans. The sample may comprise body fluids, tissue, tissue extracts, excreta, *in vitro* cultures, or environmental samples.

The invention further provides a method of detecting mycobacteria by a polymerase chain reaction wherein a DNA according the invention is used.

The invention additionally provides a recombinant protein or peptide encoded by all or part of the DNA according to the invention or a synthetic protein or peptide of the same structure; said protein or peptide being immunologically active. Antibodies to such proteins or peptides are also provided. The protein or peptide may be attached to one or more other proteins or peptides.

Such proteins or peptides may be encoded in a DNA sequence carried by a bacterium or virus, and this forms a further aspect of the invention. Such a bacterium or virus, or one or more recombinant synthetic proteins or peptides according the invention or an antibody according to the invention may be used as a vaccine, in a pharmaceutical preparation or may be optionally labelled to provide diagnostic reagent.

The diagnostic reagent may be immobilised on a solid phase, and incorporated into a diagnostic test kit The reagent or kit finds use for the diagnosis of Crohn's disease in humans or Johne's disease in animals.

### FIGURE LEGENDS

### Figure 1.

DNA was extracted from Crohn's disease-derived strains: Ben (CD1), Linda (CD2) & Dominic (CD3), M. paratuberculosis, M. avium complex serotype 2, M. avium complex serotype 5, M. kansasii, & M. phlei. DNA samples (1»g) were digested with restriction endonuclease pvuII, electrophoresed through 1% agarose, blotted onto nylon membrane, hybridised to radiolabelled probe pMB22 and autoradiographed. Molecular weight markers, in kilobases are shown on the right.

### Figure 2.

DNA was extracted from Crohn's disease - derived strains 410 (obtained from G.Gitnick) and strain H1 (obtained from J.Haagsma); and M. avium. DNA samples (1»g) were digested with restriction endonuclease PvuII, electrophoresed through 1% agarose, blotted onto nylon membrane and hybridised to radiolabelled probe pMB22, and autoradiographed. Lane 1: M. avium, lane 2: strain H1, lane 3: strain 410.

### Figure 3.

A: Restriction endonuclease map of pMB22 showing location of sequencing contigs C1 and C2 (arrows) and also location of subclones S4, S8 and S12. The exact location of subclones has not been determined, therefore their maximum extent is indicated by hatched lines. P=PvuII, X=XhoI, R=EcoRI, B=BglII, K=KpnI. Molecular weight is indicated in kilobases (Kb).
B: Restriction endonuclease maps of the loci encoding sequences contained in pMB 22 in the genomes of M. paratuberculosis and M. avium. Molecular weight is shown in Kilobases (Kb).

### Figure 4.

DNA sequence information obtained from the clone pMB22. Two contiguous lengths of sequence (contigs C1 and C2) are shown. The locations of the contigs are shown in the map of pMB22 in Fig 3A.

### Figure 5.

DNA was extracted from mycobactin-dependent disease-derived strains of M. avium, M. paratuberculosis and atypical M. avium strains. DNA samples (approx. 0.5»g) were digested with restriction endonuclease PvuII, electrophoresed through 1% agarose, blotted onto nylon membrane and hybridised to radiolabelled, pMB22 probe (A), pMB22/S4 probe (B) pMB22/S12 probe (C), and autoradiographed. DNA samples were:
M. paratuberculosis (lane 1), non-mycobactin dependent M. avium serotype 2 (lane 2), non-mycobactin-dependent armadillo-derived strain 10911 (lane 3), mycobactin-dependent goat derived strain 8589 (lane 4), mycobactin-dependent armadillo-derived strains 157 (lane 5) and 157S (lane 6), mycobactin-dependent porcine-derived strain 7941 (lane 7), mycobactin-dependent deer derived strains 8446 (lane 8) and 8438 (lane 9), mycobactin-dependent wood-pigeon derived strains 1/79 (lane 10), 5/79 (lane 11), 1/72 (lane 12) and mycobactin-dependent goat-derived strain GK27, (lane 13). Molecular weight markers are shown on the right of A.

### THE FOLLOWING EXAMPLES ILLUSTRATE THE INVENTION

### Example 1. DNA probes for the specific identification of mycobacteria.

DNA probes were derived as follows. Genomic DNA from a strain of M. paratuberculosis isolated from a human CD patient [6] was digested with Bam H1, ligated into the plasmid vector pGEM-1 (Promega-Biotech), and used to transform E. coli DH 1 cells using standard techniques (Maniatis et al. 1982. Molecular Cloning: Laboratory Manual). Greater than 20,000 ampicillin resistant recombinant clones were obtained. Insert size in plasmid DNA from 12 randomly selected clones (pMB1 to 12) ranged from 180bp to 4200 bp. This library was screened by hybridisation with hexanucleotide primed, radiolabelled total genomic DNA probes from M. paratuberculosis strain Ben, M. paratuberculosis (ATCC 19698), and M. kansasii (TMC 1201) Hybridising colonies were identified by autoradiography. Approximately 5% of clones hybridised to both M. paratuberculosis (ATCC 19698) and M. paratuberculosis (strain Ben) and 1% to M. kansasii. Forty eight clones were selected as giving either a differential hybridisation signal with DNA from the three sources, or a similar signal with DNA from the three sources, and gridded onto an ordered array. The ordered array was again hybridised with radiolabelled genomic DNA from M. paratuberculosis strain Ben, M. paratuberculosis (ATCC 19698), M. avium complex serotype 2 (Caddigg 16741), M. avium complex serotype 5 (25546-759) M. kansasii (TMC 1201) and M. phlei (NCIB 8573). Clones designated pMB13 to 24 were selected as showing either strong hybridisation to all DNA samples, or demonstrating differential hybridisation between the DNA samples.

### Example 2 - DNA probes for 16s ribosomal RNA

Probes encoding ribosomal RNA sequences were isolated by screening the M. paratuberculosis strain Ben genomic library with radiolabelled complementary DNA (cDNA) prepared from total RNA extracted from M. avium serotype 2 cells. Cells were lysed by sonication for 80 seconds in 4M guanidinium isothiocyanate and RNA purified by centrifugation through 5.7M caesium chloride and precipitated with 70% ethanol. Radiolabelled cDNA was prepared by incubating 1»g of purified RNA with reverse transcriptase, using random hexanucleotides to prime cDNA synthesis. The radiolabelled probe was hybridised to the bacterial colonies in situ. Autoradiography revealed hybridising clones which were picked and plasmid DNA extracted. This was radiolabelled and used to probe Northern blots of M. avium total RNA. A clone designated pMBr16 was found to hybridise strongly to 16S ribosomal RNA on Northern analysis.

### Example 3 - DNA probes for M.bovis and M.Leprae

In a further example of the preparation and use of DNA probes for the recognition and differentiation of a specific mycobacterial strain, DNA from Mycobacterium bovis (BCG) strain Glaxo was digested with Bam H1, ligated to vector pGEM-1 and used to transform E.Coli DH1 cells by standard techniques as before. The resulting genomic DNA library was probed with radiolabelled M. bovis genomic DNA and strongly hybridising clones designated pMBCG13-24 picked. Similarly, genomic DNA was extracted from two nude mouse isolates of Mycobacterium leprae obtained from lepromatous leprosy patient tissue and a genomic library of several thousand clones prepared. Twelve randomly selected clones containing inserts and designated pML1-12 were isolated.

### Example 4 - The use of mycobacterial DNA probes

The precise identification and differentiation between mycobacterial strains and species is in general performed by the isolation of genomic DNA from target bacteria, digestion with restriction endonucleases, electrophoresis, and Southern blotting using one of the mycobacterial DNA probes radiolabelled as described. Autoradiography reveals banding patterns that are examined for restriction fragment length polymorphisms (RFLPs) but dot blot analyses and non radioactive revealing systems as well as PCR amplification may also be employed. RFLPs are also used to measure DNA base substitution between strains and species.

Differentiation between M. paratuberculosis and M. avium complex. DNA from M. paratuberculosis ATCC 19698, M. paratuberculosis strain Ben, and M. avium serotype 2 (Caddig 16741), was digested with restriction endonucleases Bam H1, EcoR1, Bst 1, Ava 1, pst 1, Hinf 1, Hae III, Sau 3A, Hinc 1, Pvu 11, Pvu 1 and Taq 1, electrophoresed, blotted and probed with radiolabelled clones, pMB7, pMB12, pMB16, pMB17, pMB19, pMB20, pMB21, pMB22, and examined for RFLPs that distinguished between the DNA samples. (The derivation of clone pMB22 which contains the insertion sequence ISM-1 of the invention, is described in example 5). Several RFLPs were found to distinguish between the M. avium and the M. paratuberculosis strains [7]. However, no RFLPs were found to distinguish between M. paratuberculosis ATCC 19698 and M. paratuberculosis strain Ben including the complex banding pattern seen with pMB22 containing the ISM-1 insertion sequence [7], suggesting genetic identity between these two. The vaccine strain 18 presumed to be of M. paratuberculcsis was further shown in fact to be identical to M. avium serotype 1 [8].

Identification of Crohn's disease (CD) derived mycobacteria. Several isolates of mycobacteria from CD tissue have been thought to be related to M. paratuberculosis [6,9]. However, conventional techniques, which are incapable of distinguishing precisely between M. paratuberculosis and M. avium (particularly mycobactin-dependent M. avium) have left the identity of these CD isolates uncertain. Since M. avium complex strains can be isolated from healthy subjects, and are common environmental organisms [10], the exact identity of the CD-derived strains is critical to an evaluation of their etiological significance in CD. DNA was therefore extracted from three CD-derived strains (Ben, Linda and Dominic) [6], and also M. avium complex serotype 2, M. avium complex serotype 5, M. kansasii and m. phlei. DNA was digested with restriction endonucleases and probed with DNA clones of the invention identifying RFLPs as described that differentiated between M. paratuberculosis and M. avium complex serotype 2. Six different RFLPs were investigated. Each of the three CD-derived strains were shown to be identical to M. paratuberculosis [7] (Fig 1). These findings were supported by the use of the ISM-1 containing clone pMB22 of the invention to probe restricted DNA from two further uncharacterised human CD mycobacterial isolates, 410 & H1 obtained from two independent laboratories, of Drs Gitnik and Beaman UCLA in the U.S.A and Dr. J. Haagsma, National Veterinary Institute, Amsterdam, Netherlands respectively. Identical banding patterns were obtained showing that these further CD isolates were again ISM-1 containing M. paratuberculosis. (Fig 2).

Similar methods and DNA probes of the invention were used to examine the identity of 8 strains of M. paratuberculosis isolated from infected cows with Johne's disease. Isolates were derived independently from infected animals in the UK, France and the U.S.A., as well as 2 additional isolates from Macaque monkeys suffering from a disease resembling Johne's disease [11]. RFLP analysis with probes pMB14, pMB20, and pMB22 revealed that all strains gave identical banding patterns to those seen in Figs 1 and 2 for pMB22, demonstrating the conserved nature of the pathogen M. paratuberculosis. Probing with clone pMB22 containing insertion sequence ISM-1, revealed identical banding patterns for each strain.

Members of the M. avium complex of mycobacteria have been difficult to characterise and define by conventional techniques. We used the mycobacterial probes and methods of the invention described, to examine a range of M. avium complex strains. Several different patterns were obtained [8]. Serotypes 2,4,5,6, and 8 were found to be similar with less than 2% DNA base substitution between strain types. However, at least 6 strain type banding patterns were recognised. M. avium complex serotypes 11, 16 and 27 were found to have greater than 13% DNA base substitution between each other and the serotype 2,4,5,6,8 group. These strains were previously designated M. intracellulare or intermediate strains. We proposed from this work that M. avium complex strains giving banding patterns similar to those obtained with serotypes 2,4,5,6 and 8 to be designated M. avium and the heterogenous strains represented by serotypes 11, 16 and 27 be designated M. intracellulare complex [8]. Strain typing using the higher resolution DNA probe methods of the invention did not always correspond with conventional serotyping.

The methods and DNA probes of the invention were further used to distinguish M. bovis BCG (Glaxo) and M. tuberculosis strain H37RV. DNA from these mycobacteria was digested with Ava 1, Sma 1, Kpn 1, Pvu II and Pst 1, electrophoresed, blotted and probed with pMBr16 and pMBCG13. pMBr16 demonstrated an RFLP between Kpn 1 digests of M. bovis and M. tuberculosis DNA and pMBCG13 revealed an RFLP with Sma 1 digested DNA.

Similar techniques were applied to the characterisation of M. leprae. M. leprae DNA was cut with Pvu II electrophoresed, blotted and probed with pMBr16 and pML1. pML1 gave a banding pattern with M. leprae DNA but did not hybridise to DNA from other mycobacteria. pMBr16 gave an M. leprae banding pattern distinct from those seen with any other mycobacteia tested.

DNA was extracted from a range of mycobacteria, digested with Pvu II, electrophoresed, blotted, and probed with radiolabelled clone pMBr16 and other clones. The results demonstrated that each mycobacterial species gave a distinct and identifiable DNA banding pattern.

The use of cloned DNA probes of the invention for the specific identification of mycobacteria is also applicable to DNA extracts of tissues infected in vivo with mycobacteria. To demonstrate this, we extracted mouse liver infected with mycobacterium lepraemurium using methods we developed to extract mycobacterial DNA [8]. Extracted DNA was cut with Pvu II electrophoresed, blotted and probed with pMB12, pMB16, pMB20 and pMB22. Specific banding patterns were obtained for M. lepraemurium from mouse liver which showed this organism to be closely related to M. avium having an approximate 2% base substitution between it and M. avium complex serotype 2 [8].

### Example 5. The identification, cloning and characterisation of mycobacterial insertion sequences (ISM) of the invention.

Insertion sequences and transposons are short repetetive pieces of DNA within genomic DNA, which may be mobile in response to appropriate environmental conditions. They exert their biological effects either through the components which they encode or by their influence on neighbouring host genes, or host genes into which they become inserted. These elements occur widely in nature and have been identified in some bacteria, yeasts, plants, insects and animals [12]. They have not hitherto been identified in mycobacteria.

A major aspect of the present invention comprises the identification, for the first time, of a novel family of related insertion sequences in disease isolates of mycobacteria. This family of insertion sequencies is designated ISM, and an example of ISM (ISM-1) we have cloned and characterised in detail. Members of the ISM family of mycobacterial insertion sequences of the invention are identified by sharing partial sequence homology and hybridising at low stringency with ISM-1 in the clone pMB22 deposited with the National Collections of Industrial and Marine Bacteria, number 12461 Aberdeen, Scotland, U.K. All or part of the DNA or RNA encoded by members of the ISM family and all or part of the proteins and peptides encoded by ISM or mutagenised or altered in expression by ISM are applicable to use in a novel range of diagnostics for disease-causing mycobacteria containing ISM. ISM of the invention are also used by specific deletion or transfection, to generate mutant or recombinant bacteria or viruses for the provision of novel vaccines, agents for immunotherapy, or diagnostics.

Genomic DNA from a Crohn's disease isolate of Mycobacterium paratuberculosis was used to generate a genomic DNA recombinant library using the plasmid vector pGEM-1 and E. coli DH1 cells by standard techniques as described. This library was screened with radiolabelled DNA probes derived from M. paratuberculosis, M. kansasii and M. avium. There was only one clone identified which hybridised strongly to M. paratuberculosis but only weakly to M. avium and M. kansassi DNA and we designated this clone pMB22. This was picked and found to contain an insert of about 5kb. This recombinant DNA fragment was excised, purified, digested with further restriction endonucleases, and sub-cloned into either the plasmid vector pGEM-1, or the M13 phage vectors mp18 and mp19. Resulting clones were hybridised to M. paratuberculosis DNA and strongly positive clones were identified. The location of selected subclones, within the 5kb insert in pMB22, some of which hybridised strongly with M. paratuberculosis DNA, are shown (Fig 3A). E.Coli strain DH1 harbouring pMB22 was deposited on 24th April, 1987 under Budapest treaty conditions, with the National Collections of Industrial and Marine Bacteria, Aberdeen, Scotland, U.K. where it was given the deposit number NCIB 12461.

### Example 6. Insertion sequence ISM-1 in M. paratuberculosis.

This section cites evidence showing that ISM-1 in M. paratuberculosis are insertion sequences and that these segregate with the pathogenicity of this organism.

M. paratuberculosis DNA was cut with Bam H1, Sst1, EcoR1 and Bst1, electrophoresed, blotted, and hybridised to pMB22 and its subclones pMB22/S4 and pMB22/S12. In each case approximately 8 strongly hybridising bands were obtained. When this procedure was repeated using the restriction endonucleases Alu1, Ava1, Pst1 PvuII and Pvu1, approximately 16 strongly hybridising bands were seen with pMB22 and its subclone S4, while a subset of only 8 of these bands hybridised to the subclone of pMB22, S12. Digestion of M. paratuberculosis genomic DNA with Hinf1 produced 2 bands hybridising strongly with pMB22 and pMB22/S4, only one of which also hybridised with pMB22/S12. pMB22 and its subclones S4 and S12 were then used to probe Southern blots of Bam H1 restricted genomic DNA obtained from two laboratory strains of M. avium serotype 2 (Caddigg 16741) and serotype 5 (25546-759) [7]. A single band was found with both M. avium strains using pMB22 probe. pMB22/S12 did not hybridise to M. avium serotype 2 DNA, and pMB22/S4 hybridised only very weakly to a single band.

These results, taken together with those in example 5, indicate that pMB22 contains a sequence that is present in approximately 8 copies dispersed in the genome of M. paratuberculosis but absent in M. avium serotype 2. The enzymes giving 8 hybridising bands must not cut the insertion sequence contained within clone pMB22 and each band therefore represents a different genomic location of the ISM insertion elements. Enzymes such as PvuII cut the element once and therefore produce two fragments for each genomic copy. The sequence pMB22/S12 must be derived from one of the Pvu II fragments, whereas pMB22/S4 spans the PvuII site. The enzyme Hinf 1 must have at least 3 sites within the insertion element, and therefore each genomic copy of ISM is cleaved to give the same two internal ISM fragments, plus heterogenous ISM fragments containing flanking DNA. A detailed restriction endonuclease map of pMB22 was derived and is shown (Fig 3A).

The data presented show that the M. paratuberculosis and M. avium complex strains tested are very closely related, exhibiting less than 1% DNA base substitution between the two species [7,8]. However, the identical nature of M. paratuberculosis strains isolated from a wide variety of geographical sources and disease hosts, suggests that M. paratuberculosis is a conserved specific pathogen causing Johne's disease in cattle, goats, and at least one species of primate, and possibly causing some cases of Crohn's disease in humans. This would suggest there are specific genetic changes that characterise M. paratuberculosis, confering pathogenicity and enabling it to cause these diseases. The tight segregation of ISM-1 insertion sequences to M. paratuberculosis and the specific absence of ISM-1 from most laboratory strains of M. avium examined, would make IMS-1 a strong candidate sequence responsible for the genetic changes in M. paratuberculosis endowing its pathogenicity.

The organisation of genomic DNA flanking the insertion sequence ISM-1 of the invention, identified within clone pMB22 and M. paratuberculosis, was examined with pMB22 and subclones, S4, S8, and S12 and the results compared with similar studies on M. avium genomic DNA. The maps are shown (Fig. 3B). Genomic DNA flanking ISM-1 in pMB22 showed no changes detectable between M. avium and M. paratuberculosis, indicating a similar level of base substitution between DNA in this region as elsewhere in the genome. Since M. paratuberculosis and M. avium are evolutionarily very close, the presence of the repetitive element ISM-1 in M. paratuberculosis is compatible with ISM-1 being recently acquired by M. paratuberculosis causing a divergence from M. avium. Alternatively the sequence ISM-1 may have been deleted from M. avium during evolution causing its divergence from M. paratuberculosis. Since the latter proposal would require precise excision and deletion of each copy of ISM-1 from the M. paratuberculosis genome, an extremely unlikely event, the former explanation is more plausible. The repetitive sequence ISM-1 would form an example of the genetic elements known as Insertion Sequences [12]. The DNA sequence of ISM-1 was determined by single-stranded M13 sequencing and is shown (Fig 4).

### Example 7. ISM related sequences in other mycobacteria.

This section demonstrates the presence of sequences in other pathogenic mycobacteria which are related to ISM-1 by sharing homology, and which are identified by hybridisation with pMB22 or its subclones under appropriate experimental conditions. As with ISM-1, other members of the ISM family appear to confer pathogenicity on the mycobacterial host.

DNA from a collection of mycobactin-dependent M. avium strains isolated from diseases in wood-pigeon, deer, goat, pig and armadillo and also M. avium serotype 2 and M. paratuberculosis were hybridised with pMB22 and its subclones. Hybridisation of mycobactin-dependent M. avium strains with pMB22 gave banding patterns similar to those obtained with DNA from M. avium serotype 2, but also showed a series of additional bands, not present in M. avium serotype 2 (Fig 5A). When washed at high stringency the signal from most of these additional bands was selectively removed. When representative DNA samples were probed with subclone pMB22/S4, these additional bands hybridised strongly, but only one faint band was obtained with M. avium serotype 2, derived from flanking DNA (Fig 5B). The subclone pMB22/S12 hybridised ISM-1 in M. paratuberculosis DNA but only to a single band in samples 157R and 157S, armadillo-derived mycobactin-dependent strains (Fig 5C). The non-mycobactin dependent armadillo-derived strain of M. paratuberculosis 10911 did not hybridise either to pMB22/S4 or pMB22/S12. When these DNA samples were probed with an unrelated M. paratuberculosis DNA probe, very similar or identical banding patterns were obtained from all strains.

These results indicate that pathogenic mycobactin-dependent strains of M. avium contain insertion sequences related to ISM-1 in the pathogen M. paratuberculosis. The strains 157R and 157S contain an insertion sequence closely related to ISM-1 since it hybridised to both pMB22/S4 and pMB22/S12. The insertion sequence present in the deer, goat, wood pigeon and porcine mycobacterial isolates are less closely related to ISM-1 and do not contain sequences homologous to pMB22/S12. The banding patterns were similar for some of the different strains in this group and the insertion sequence in these strains has been designated ISM-2.

Further unselected M. avium strains were investigated for the presence of insertion sequences of the ISM family. Several strains were found to contain copies of ISM-2 or related insertion sequences, but to date no other mycobacterial strains containing ISM-1 have been identified. Two further pathogens have been found to contain ISM-related insertion sequences. DNA from the leprosy bacillus M. leprae contains a sequence that hybridised to pMB22/S4. DNA from the rat leprosy bacillus Mycobacterium lepraemurium contained a sequence that hybridised to pMB22/S4, but not to pMB22/S12.

These results show that insertion sequences related to ISM-1 are found in a number of mycobacterial pathogens, particularly within the M. avium complex including those isolated from AIDS patients. We have screened more than 20 further mainly saprophytic and environmental mycobacterial species and strains and found none to contain insertion sequences of the ISM family. ISM appears to segregate with mycobacterial pathogenicity.

### APPLICATIONS AND USES OF THE INVENTION

Cloned DNA probes of the invention from recombinant DNA libraries derived from specific mycobacteria or synthetic DNA sequences based on information from such libraries and selected for mycobacterial strain or phenotypic specificity, are used in the specific identification of mycobacterial DNA or RNA and the differentiation of mycobacterial from other microbial sequences. Such specific mycobacterial DNA probes are also used for the precise differentiation between mycobacterial strains and species and the recognition or prediction of phenotypic properties such as drug sensitivity and pathogenicity.

In addition, DNA or RNA probes or synthetic oligonucleotides derived from all or part of the sequence ISM-1 (in pMB22 deposited with NCIB No.12461), ISM-2, and other members of the ISM family of mycobacterial insertion sequences of the invention sharing partial homology with ISM-1, are used as probes for the specific detection of potentially pathogenic strains of mycobacteria. These include M. paratuberculosis, pathogenic forms of M. avium including those involved in AIDS superinfections, and other pathogenic mycobacteria containing ISM sequences including M. leprae.

Precise identification and differentiation of mycobacteria as well as the specific detection of ISM-containing pathogenic strains, is applied to environmental samples such as water, animal and human food stuffs, and soil. Such applications are useful in the prevention and public health containment of mycobacterial diseases in humans and animals. Tests using the methods and probes of the invention are also applied to medical and veterinary samples of tissue, tissue extracts, body fluids, including milk, and excreta, as well as to the accelerated identification and characterisation of mycobacteria grown in in-vitro cultures, and to the improvement of such culture conditions. These applications are useful in the diagnosis of mycobacterial diseases such as Johne's disease in cattle and other animals, mycobacterial diseases in zoo animals and birds, human mycobacterial diseases such as leprosy, M.avium infections, AIDS mycobacterial superinfections, and to human Crohn's disease.

In methods used in the tests, DNA probes are single or double-stranded or are RNA transcripts, and may be unlabelled or labelled with radioactive, biotinyl, fluorescent, enzyme immunological or other revealing agents or methods. Probes representing all or part of ISM are used in solution phase systems or bound to solid phase supports such as cellulose, nylon, nitrocellulose, plastic, or gel systems, in the form of beads, tubes, or other matrix. Samples are treated to release mycobacterial DNA or RNA. Mechanical disruption, and/or sonication and/or enzymic treatments and/or heat treatment, and/or chemical treatments are used. Treated samples are mixed with the probe (boiled, if double-stranded) in a solution designed to allow hybridisation and prevent nuclease action. Hybridisation of specific probes to target DNA or RNA present in the sample is achieved by standard procedures.

Detection of hybrids is by a number of methods. Hydroxyapatite (HAP) may be added to bind double-stranded DNA or RNA, and the HAP is then washed to remove unhybridised probe. Other solid matrixes are used to bind hybrid. Alternatively, unhybridised probe is removed by digestion with single-strand specific nuclease followed by precipitation of hybrid by trichloroacetic acid, ethanol or other substance. Hybrids are detected by methods dependent on the revealing method used. Unlabelled probe DNA is bound to a solid matrix and used to hybridise to the sample. Target mycobacterial DNA or RNA homologous to the probe sequence becomes bound to the matrix via the probe. The matrix is washed and target DNA or RNA released by heating or transfer to denaturing solution. The target DNA or RNA is then detected directly by the methods described above. Alternatively the DNA or RNA is first amplified by polymerase chain reactions (PCR [12]) using DNA polymerase or reverse transcriptase plus DNA polymerase together with specific oligonucleotide primers for sequences present in the probe or ISM sequence, and such primers form further aspects of the present invention. After amplification, target sequences are detected by any of the methods described above. Detection may involve differentiation between unreacted primers and primers that have been extended in the PCR reactions. In this case the primers are labelled and detection is dependent on the label used. Hybridisation of probe to unamplified or amplified target mycobacterial sequences is by standard procedures and dot blot methods or the identification of RFLPs are also used.

All or part of the mycobacterial insertion sequences of the invention including ISM-1 (in pMB22), ISM-2 and other members of the ISM family sharing partial sequence homology with ISM-1, are used to generate recombinant proteins or peptides or synthetic peptides based on ISM sequences and such derived sequences, form further aspects of the present invention. Also of use are proteins mutated or altered in expression by ISM.

Recombinant proteins and ISM derived peptides are also used in immuno and other diagnostic assays using standard techniques applicable to medical, veterinary, environmental and in-vitro culture samples for the specific detection of ISM-containing pathogenic mycobacteria. Antibodies to ISM derived proteins or peptides whether polyclonal or monoclonal for use in such diagnostic immunoassays or other systems form further aspects of the present invention. Recombinant proteins based on ISM sequences are used with or without computer-based structural studies for the rational design of specific drugs to modify or block their biological actions. Recombinant ISM proteins or peptides may themselves be used as therapeutic agents to mediate specific biological effects in vivo such as immunomodulation.

All or part of the DNA sequence of members of the ISM family may be used to engineer mutant bacteria or viruses and such mutant forms containing ISM sequences form further aspects of the present invention. These are employed as specific vaccines for the prevention of diseases caused by pathogenic mycobacteria in medical and veterinary practise. They may also be used as agents in immunotherapy or diagnostics. Proteins or peptides derived from ISM are also be used as vaccines for the same purpose.

### REFERENCES

1. Goodfellow M and Wayne LG. (1982). in The Biology of Mycobacteria. ed. Ratledge & Stanford. Academic Press. London.
2. Graham, DY, et al. (1987).
   Gastroenterol 92: 436-442.
3. Chiodini RJ, et al., (1984).
   The Cornell Veterinarian 74: 218-262.
4. Collins F.
   Int.J.Leprosy 54: 458-474.
5. Van Eden et al. (1988).
   Nature 331: 171-173.
6. Chiodini RJ et al. (1984)
   Dig.Dis.Sci. 29: 1073-1079.
7. McFadden JJ. et al. (1987)
   J.Clin.Microbiol. 25: 796-801.
8. McFadden JJ. et al. (1987).
   Mol.microbiol. 1: 283-291.
9. Collins J, et al. (1987). Gastroenterol. 92: 1352.
10. Martin CM, et al (1987).
   Am.Rev.Res.Dis. 136: 344-348.
11. McClure HM, et al. (1987).
   J.Inf.Dis. 155: 1011-1019.
12. Cullum J. (1985). in Genetics of Bacteria. ed.Scaife J. et al., Academic Press, London.
13. Scharf SJ, et al. (1987).
   Science. 233: 1076-78.

## Claims (Claims for the following Contracting State(s): AT, BE, CH, LI, DE, FR, GB, IT, LU, NL, SE)

1. The plasmid pMB22 (NCIMB 12461) containing IS900 of mycobacterial origin.

2. A host cell transformed by the plasmid of claim 1.

3. Isolated DNA consisting essentially of a mycobacterial DNA sequence having at least 70% sequence homology with any contiguous stretch of at least 20 nucleotides of the IS900 insertion sequence carried by pMB22 (NCIMB 12461).

4. Isolated DNA according to claim 3 having at least 70% sequence homology to the entire IS900 insertion sequence carried by pMB22 (NCIMB 12461).

5. Isolated DNA according to claim 4 which is the IS900 insertion sequence of pMB22 (NCIMB 12461).

6. An RNA or synthetic oligonucleotide probe based upon a DNA sequence of any one of claims 3 to 5.

7. A probe comprising a DNA according to any one of claims 3 to 5 or an RNA or synthetic oligonucleotide according to claim 6 carrying a revealing label.

8. A method of obtaining a mycobacterial Insertion Element or fragment thereof which comprises probing the DNA of a pathogenic mycobacteria with a probe according to any one of claims 3 to 7 to identify insertion sequence element or fragment thereof which segregates with mycobacterial pathogenicity, and isolating said insertion sequence element or fragment thereof.

9. A mycobacterial Insertion Element or fragment thereof obtainable from the method of claim 8 or a synthetic DNA comprising the sequence thereof or an RNA based upon said DNA sequence; optionally carrying a revealing label, for use in a method of detecting the presence or absence or a mycobacterial strain or phenotype.

10. A method for detecting the presence or absence of a mycobacterial strain or phenotype which comprises assaying a sample of mycobacterial DNA or RNA with an isolated DNA or a probe according to any one of claims 3 to 7 or 9.

11. A method according to claim 10 for the precise identification and differentiation of closely related mycobacterial strains and species.

12. A method according to claim 11 wherein the or one of the mycobacterial strain(s) is *M. paratuberculosis.*

13. A method according to claim 11 wherein the or one of the mycobacterial strain(s) is of the group *M. avium-intracellulare* or *M. avium* complex.

14. A method according to claim 11 wherein the or one of the mycobacterial strain(s) is *M. leprae.*

15. A method according to claim 11 wherein the or one of the mycobacteria cause AIDS superinfections or atypical tuberculosis.

16. A method according to claim 11 wherein the phenotype is pathogenicity or drug resistance.

17. A method according to claim 11 wherein the mycobacterial strain causes Johne's disease in animals or Crohn's disease in humans.

18. A method according to any one of claims 11 to 17 wherein the sample comprises body fluids, tissue, tissue extracts, excreta, *in vitro* cultures, or environmental samples.

19. A method of detecting mycobacteria by a polymerase chain reaction wherein a DNA according to any one of claims 3 to 5, or 9 is used.

20. A recombinant protein or peptide encoded by all or part of the DNA according to any one of claims 3 to 5, 7 or 9 or a synthetic protein or peptide of the same structure; said protein or peptide being immunologically active.

21. An antibody to a protein or peptide according to claim 20.

22. A recombinant protein or peptide comprising a protein or peptide according to claim 20 attached to one or more other proteins or peptides.

23. A bacterium or virus carrying a DNA sequence encoding a recombinant protein or peptide according to claim 20 or 22.

24. A vaccine against mycobacterial disease in animals or humans comprising a bacterium or virus according to claim 23, one or more recombinant synthetic proteins or peptides according to claim 20 or 22, or an antibody according to claim 21.

25. A pharmaceutical preparation comprising, as active ingredient, a bacterium or virus according to claim 23, one or more recombinant synthetic proteins or peptides according to claim 20 or 22 or an antibody according to claim 21.

26. An optionally labelled diagnostic reagent which comprises an isolated DNA or a probe according to any one of claims 3 to 7 or 9, a bacterium or virus according to claim 23, a recombinant or synthetic protein or peptide according to claim 20 or 22 or an antibody according to claim 21.

27. An optionally labelled diagnostic reagent according to claim 26 immobilised on a solid phase.

28. A diagnostic test kit comprising, as one component, an optionally labelled reagent according to claim 26 or 27.

29. A diagnostic reagent according to claim 26 or 27 or a test kit according to claim 28 for the diagnosis of Crohn's disease in humans or Johne's disease in animals.

## Claims (Claims for the following Contracting State(s): ES, GR)

1. A method of obtaining a mycobacterial Insertion Element or fragment thereof which comprises probing the DNA of a pathogenic mycobacteria with a probe, said probe comprising:
(a) isolated DNA consisting essentially of a mycobacterial DNA sequence having at least 70% sequence homology with any contiguous stretch of at least 20 nucleotides of the IS900 insertion sequence carried by pMB22 (NCIMB 12461);
(b) isolated DNA having at least 70% sequence homology to the entire IS900 insertion sequence carried by pMB22 (NCIMB 12461);
(c) isolated DNA which is the IS900 insertion sequence of pMB22 (NCIMB 12461);
(d) an RNA or synthetic oligonucleotide probe based upon a DNA sequence of any one of (a) to (c); or
(e) a probe comprising a DNA according to any one of (a) to (c) or an RNA or synthetic oligonucleotide according to (d) carrying a revealing label;
to identify an insertion sequence element or fragment thereof which segregates with mycobacterial pathogenicity, and isolating said insertion sequence element or fragment thereof.

2. A method for detecting the presence or absence of a mycobacterial strain or phenotype which comprises assaying a sample of mycobacterial DNA or RNA with a probe comprising:
(a) isolated DNA consisting essentially of a mycobacterial DNA sequence having at least 70% sequence homology with any contiguous stretch of at least 20 nucleotides of the IS900 insertion sequence carried by pMB22 (NCIMB 12461);
(b) isolated DNA having at least 70% sequence homology to the entire IS900 insertion sequence carried by pMB22 (NCIMB 12461);
(c) isolated DNA which is the IS900 insertion sequence of pMB22 (NCIMB 12461);
(d) an RNA or synthetic oligonucleotide probe based upon a DNA sequence of any one of (a) to (c); or
(e) a probe comprising a DNA according to any one of (a) to (c) or an RNA or synthetic oligonucleotide according to (d) carrying a revealing label;
or a mycobacterial insertion element or fragment thereof obtainable by the method of claim 1.

3. A method according to claim 2 for the precise identification and differentiation of closely related mycobacterial strains and species.

4. A method according to claim 3 wherein the or one of the mycobacterial strain(s) is *M. paratuberculosis.*

5. A method according to claim 3 wherein the or one of the mycobacterial strain(s) is of the group *M. avium-intracellulare* or *M. avium* complex.

6. A method according to claim 3 wherein the or one of the mycobacterial strain(s) is *M. leprae.*

7. A method according to claim 3 wherein the or one of the mycobacteria cause AIDS superinfections or atypical tuberculosis.

8. A method according to claim 3 wherein the phenotype is pathogenicity or drug resistance.

9. A method according to claim 3 wherein the mycobacterial strain causes Johne's disease in animals or Crohn's disease in humans.

10. A method according to any one of claims 3 to 9 wherein the sample comprises body fluids, tissue, tissue extracts, excreta, *in vitro* cultures, or environmental samples.

11. A method of detecting mycobacteria by a polymerase chain reaction wherein a fragment obtainable by the method of claim 1 is used.

## Patentansprüche (Patentansprüche für folgende(n) Vertragsstaat(en): AT, BE, CH, LI, DE, FR, GB, IT, LU, NL SE)

1. Das Plasmid pMB22 (NCIMB 12461), enthaltend die IS900 mycobakteriellen Ursprungs.

2. Wirtszelle, die mit dem Plasmid aus Anspruch 1 transformiert ist.

3. Isolierte DNA, im wesentlichen bestehend aus einer Mycobakterien-DNA-Sequenz mit mindestens 70 % Sequenzhomologie zu jedwedem zusammenhängenden Abschnitt von mindestens 20 Nukleotiden der von pMB22 (NCIMB 12461) getragenen Insertionssequenz IS900.

4. Isolierte DNA nach Anspruch 3 mit mindestens 70 % Sequenzhomologie zu der gesamten von pMB22 (NCIMB 12461) getragenen Insertionssequenz IS900.

5. Isolierte DNA nach Anspruch 4, welche die Insertionssequenz IS900 von pMB22 (NCIMB 12461) ist.

6. RNA- oder synthetische Oligonukleotidsonde, basierend auf einer DNA-Sequenz nach mindestens einem der Ansprüche 3 bis 5.

7. Sonde, welche DNA nach mindestens einem der Ansprüche 3 bis 5 oder RNA oder ein synthetisches Oligonukleotid nach Anspruch 6, welche(s) eine nachweisbare Markierung trägt, umfaßt.

8. Verfahren zur Gewinnung eines mycobakteriellen Insertionselementes oder eines Fragmentes davon, das die Untersuchung der DNA eines pathogenen Mycobakteriums mit einer Sonde gemäß mindestens einem der Ansprüche 3 bis 7, um ein Insertionssequenzelement oder ein Fragment davon zu identifizieren, welches mit der Mycobakterien-Pathogenität einhergeht, und das Isolieren des Insertionssequenzelements oder des Fragmentes davon umfaßt.

9. Mycobakterien-Insertionselement oder Fragment davon, erhältlich durch das Verfahren von Anspruch 8, oder eine synthetische DNA, die die Sequenz davon beinhaltet, oder eine auf der DNA-Sequenz basierende RNA; welche(s) gegebenenfalls eine nachweisbare Markierung trägt, zur Verwendung in einem Verfahren zum Nachweis der Gegenwart oder Abwesenheit eines Mycobakterienstammes oder -Phänotyps.

10. Verfahren zum Nachweis der Gegenwart oder Abwesenheit eines Mycobakterien-Stammes oder -Phänotyps, das den Assay einer Probe von Mycobakterien-DNA oder -RNA mit einer isolierten DNA oder einer Sonde nach mindestens einem der Ansprüche 3 bis 7 oder 9 umfaßt.

11. Verfahren nach Anspruch 10 für die präzise Identifikation und Differenzierung nahe verwandter Mycobakterienstämme und -arten.

12. Verfahren nach Anspruch 11, worin der oder einer der Mycobakterienstämme *M. paratuberculosis* ist.

13. Verfahren nach Anspruch 11, worin der oder einer der Mycobakterienstämme aus der Gruppe *M. avium-intracellulare* oder dem *M. avium*-Komplex ist.

14. Verfahren nach Anspruch 11, worin der oder einer der Mycobakterienstämme *M. leprae* ist.

15. Verfahren nach Anspruch 11, worin das oder eines der Mycobakterien AIDS-Superinfektionen oder atypische Tuberkulose verursacht.

16. Verfahren nach Anspruch 11, worin der Phänotyp in Pathogenität oder Arzneimittel-Resistenz besteht.

17. Verfahren nach Anspruch 11, worin der Mycobakterienstamm die John-Krankheit bei Tieren oder die Crohn-Krankheit bei Menschen verursacht.

18. Verfahren nach mindestens einem der Ansprüche 11 bis 17, worin die Probe Körperflüssigkeiten, Gewebe, Gewebeextrakte, Exkrete, *in vitro*-Kulturen oder Umwelt-Proben umfaßt.

19. Verfahren zum Nachweis von Mycobakterien durch eine Polymerase-Kettenreaktion, worin eine DNA nach mindestens einem der Ansprüche 3 bis 5 oder 9 verwendet wird.

20. Rekombinantes Protein oder Peptid, codiert von der gesamten oder einem Teil der DNA nach mindestens einem der Ansprüche 3 bis 5, 7 oder 9, oder ein synthetisches Protein oder Peptid derselben Struktur; wobei das Protein oder Peptid immunologisch aktiv ist.

21. Antikörper gegen ein Protein oder Peptid nach Anspruch 20.

22. Rekombinantes Protein oder Peptid, umfassend ein Protein oder Peptid nach Anspruch 20, das an ein oder mehrere andere Proteine oder Peptide geknüpft ist.

23. Bakterium oder Virus, das eine für ein rekombinantes Protein oder Peptid nach Anspruch 20 oder 22 codierende DNA-Sequenz trägt.

24. Impfstoff gegen eine Mycobakterien-Erkrankung bei Tieren oder Menschen, umfassend ein Bakterium oder Virus nach Anspruch 23, ein oder mehrere rekombinante synthetische Proteine oder Peptide nach Anspruch 20 oder 22 oder einen Antikörper nach Anspruch 21.

25. Pharmazeutische Zusammensetzung, umfassend als Wirkstoff ein Bakterium oder Virus nach Anspruch 23, ein oder mehrere rekombinante synthetische Proteine oder Peptide nach Anspruch 20 oder 22 oder einen Antikörper nach Anspruch 21.

26. Gegebenenfalls markiertes diagnostisches Reagenz, welches eine isolierte DNA oder eine Sonde nach mindestens einem der Ansprüche 3 bis 7 oder 9, ein Bakterium oder Virus nach Anspruch 23, ein rekombinantes oder synthetisches Protein oder Peptid nach Anspruch 20 oder 22 oder einen Antikörper nach Anspruch 21 umfaßt.

27. Gegebenenfalls markiertes diagnostisches Reagenz nach Anspruch 26, das auf einer Festphase immobilisiert ist.

28. Diagnostisches Testkit, umfassend, als eine Komponente, ein gegebenfalls markiertes diagnostisches Reagenz nach Anspruch 26 oder 27.

29. Diagnostisches Reagenz nach Anspruch 26 oder 27 oder ein Testkit nach Anspruch 28 für die Diagnose der Crohn-Krankheit bei Menschen oder der John-Krankheit bei Tieren.

## Patentansprüche (Patentansprüche für folgende(n) Vertragsstaat(en): ES, GR)

1. Verfahren zur Gewinnung eines Mycobakterien-Insertionselementes oder eines Fragmentes davon, welches die Untersuchung der DNA eines pathogenen Mycobakteriums mit einer Sonde beinhaltet, wobei die Sonde folgendes umfaßt:
(a) isolierte DNA, im wesentlichen bestehend aus einer mycobakteriellen DNA-Sequenz mit mindestens 70% Sequenzhomologie zu jedwedem zusammenhängenden Abschnitt von mindestens 20 Nukleotiden der von pMB22 (NCIMB 12461) getragenen Insertionssequenz IS900;
(b) isolierte DNA mit mindestens 70 % Sequenzhomologie zu der gesamten von pMB22 (NCIMB 12461) getragenen Insertionssequenz: IS900;
(c) isolierte DNA, welche die Insertionssequenz IS900 von pMB22 (NCIMB 12461) ist;
(d) RNA- oder synthetische Oligonukleotidsonde, basierend auf einer DNA-Sequenz von einer von (a) bis (c); oder
(e) Sonde, welche DNA gemäß mindestens einer von (a) bis (c) oder RNA oder ein synthetisches Oligonukleotid gemäß (d), welche(s) eine nachweisbare Markierung trägt, umfaßt;
um ein Insertionssequenzelement oder ein Fragment davon zu identifizieren, welches mit der Mycobakterien-Pathogenität einhergeht, und das Isolieren des Insertionssequenzelements oder des Fragmentes davon umfaßt.

2. Verfahren zum Nachweis der Gegenwart oder Abwesenheit eines Mycobakterien-Stammes oder -Phänotyps, welches den Assay einer Probe von Mycobakterien-DNA oder -RNA mit einer Sonde, umfassend
(a) isolierte DNA, im wesentlichen bestehend aus einer Mycobakterien-DNA-Sequenz mit mindestens 70% Sequenzhomologie zu jedwedem zusammenhängenden Abschnitt von mindestens 20 Nukleotiden der von pMB22 (NCIMB 12461) getragenen Insertionssequenz IS900;
(b) isolierte DNA mit mindestens 70 % Sequenzhomologie zu der gesamten von pMB22 (NCIMB 12461) getragenen Insertionssequenz IS900;
(c) isolierte DNA, welche die Insertionssequenz IS900 von pMB22 (NCIMB 12461) ist;
(d) RNA- oder synthetische Oligonukleotidsonde, basierend auf einer DNA-Sequenz von einer von (a) bis (c); oder
(e) Sonde, welche DNA gemäß mindestens einer von (a) bis (c) oder RNA oder ein synthetisches Oligonukleotid gemäß (d), welche(s) eine nachweisbare Markierung trägt, umfaßt;
oder einem Mycobakterien-Insertionselement oder einem Fragment davon, erhältlich durch das Verfahren von Anspruch 1, umfaßt.

3. Verfahren nach Anspruch 2 für die präzise Identifizierung und Differenzierung nahe verwandter Mycobakterienstämme und -arten.

4. Verfahren nach Anspruch 3, worin der oder einer der Mycobakterienstämme *M. paratuberculosis* ist.

5. Verfahren nach Anspruch 3, worin der oder einer der Mycobakterienstämme aus der Gruppe *M. avium-intracellulare* oder dem *M. avium*-Komplex ist.

6. Verfahren nach Anspruch 3, worin der oder einer der Mycobakterienstämme *M. leprae* ist.

7. Verfahren nach Anspruch 3, worin das oder eines der Mycobakterien AIDS-Superinfektionen oder atypische Tuberkulose verursacht.

8. Verfahren nach Anspruch 3, worin der Phänotyp in Pathogenität oder Arzneimittel-Resistenz besteht.

9. Verfahren nach Anspruch 3, worin der Mycobakterienstamm die John-Krankheit bei Tieren oder die Crohn-Krankheit bei Menschen verursacht.

10. Verfahren nach mindestens einem der Ansprüche 3 bis 9, worin die Probe Körperflüssigkeiten, Gewebe, Gewebeextrakte, Exkrete, *in vitro*-Kulturen oder Umwelt-Proben umfaßt.

11. Verfahren zum Nachweis von Mycobakterien durch eine Polymerase-Kettenreaktion, worin ein durch das Verfahren von Anspruch 1 erhältliches Fragment verwendet wird.

## Revendications (Revendications pour l'(les) Etat(s) contractant(s) suivant(s): AT, BE, CH, LI, DE, FR, GB, IT, LU, NL, SE)

1. Plasmide pMB22 (NCIMB 12461) contenant la séquence d'insertion IS900 d'origine mycobactérienne.

2. Cellule hôte transformée par le plasmide de la revendication 1.

3. ADN isolé essentiellement constitué d'une séquence d'ADN mycobactérien ayant au moins 70 % d'homologie de séquence avec un quelconque segment contigu d'au moins 20 nucléotides de la séquence d'insertion IS900 portée par pMB22 (NCIMB 12461).

4. ADN isolé selon la revendication 3, ayant au moins 70 % d'homologie de séquence avec la séquence d'insertion IS900 entière portée par pMB22 (NCIMB 12461).

5. ADN isolé selon la revendication 4, qui est la séquence d'insertion IS900 de pMB22 (NCIMB 12461).

6. Sonde d'ARN ou d'oligonucléotide synthétique sur la base d'une séquence d'ADN de l'une quelconque des revendications 3 à 5.

7. Sonde comprenant un ADN selon l'une quelconque des revendications 3 à 5 ou un ARN ou un oligonucléotide, synthétique selon la revendication 6 portant un marqueur de détection.

8. Procédé d'obtention d'une Elément d'Insertion mycobactérien ou d'un fragment de celui-ci, qui comprend la recherche de l'ADN d'une mycobactérie pathogène avec une sonde selon l'une quelconque des revendications 3 à 7 pour identifier un élément de la séquence d'insertion ou un fragment de celui-ci qui se sépare avec la pathogénie mycobactériene, et l'isolement dudit élément de la séquence d'insertion ou d'un fragment de celui-ci.

9. Elément d'insertion mycobactérien ou fragment de celui-ci, pouvant être obtenu par le procédé de la revendication 8, ou ADN synthétique comprenant la séquence de celui-ci ou un ARN basé sur ladite séquence d'ADN ; portant éventuellement un marqueur de détection, pour utilisation dans un procédé de détection de la présence ou de l'absence d'une souche ou d'un phénotype mycobactérien.

10. Procédé pour détecter la présence ou l'absence d'une souche ou d'un phénotype mycobactérien, qui comprend l'essai d'un échantillon d'ADN ou d'ARN mycobactérien avec un ADN isolé ou une sonde selon l'une quelconque des revendications 3 à 7 ou 9.

11. Procédé selon la revendication 10 pour l'identification et la différentiation précises de souches et espèces mycobactériennes étroitement apparentées.

12. Procédé selon la revendication 11, dans lequel la souche ou l'une des souches mycobactérienne(s) est *M. paratuberculosis.*

13. Procédé selon la revendication 11, dans lequel la souche ou l'une des souches mycobactérienne(s) fait partie du groupe du complexe *M. avium-intracellulare* ou *M. avium.*

14. Procédé selon la revendication 11, dans lequel la souche ou l'une des souches mycobactériennes(s) est *M. leprae.*

15. Procédé selon la revendication 11, dans lequel la mycobactérie ou l'une des mycobactéries provoquent des surinfections liées au SIDA ou une tuberculose atypique.

16. Procédé selon la revendication 11, dans lequel le phénotype est la pathogénie ou la résistance aux médicaments.

17. Procédé selon la revendication 11, dans lequel la souche mycobactérienne provoque la maladie de Johne chez les animaux ou la maladie de Crohn chez l'homme.

18. Procédé selon l'une quelconque des revendications 11 à 17, dans lequel l'échantillon comprend des fluides corporels, un tissu, des extraits tissulaires, des excreta, des cultures *in vitro* ou des échantillons de l'environnement.

19. Procédé de détection de mycobactéries par une réaction en chaîne par polymérase, dans lequel on utilise un ADN selon l'une quelconque des revendications 3 à 5, ou 9.

20. Protéine ou peptide recombinant(e), codé(e) par tout ou partie de l'ADN selon l'une quelconque des revendications 3 à 5, 7 ou 9, ou protéine ou peptide synthétique de la même structure ; ladite protéine ou ledit peptide étant immunologiquement actif.

21. Anticorps contre une protéine ou un peptide selon la revendication 20.

22. Protéine ou peptide recombinant(e), comprenant une protéine ou un peptide selon la revendication 20 attaché(e) à un(e) ou plusieurs autres protéines ou peptides.

23. Bactérie ou virus portant une séquence d'ADN codant pour une protéine ou un peptide recombinant(e) selon la revendication 20 ou 22.

24. Vaccin contre une maladie mycobactérienne chez les animaux ou les humains, comprenant une bactérie ou un virus selon la revendication 23, un(e) ou plusieurs protéines ou peptides synthétiques recombinants selon la revendication 20 ou 22, ou un anticorps selon la revendication 21.

25. Préparation pharmaceutique comprenant, en tant qu'ingrédient actif, une bactérie ou un virus selon la revendication 23, un(e) ou plusieurs protéines ou peptides synthétiques recombinants selon la revendication 20 ou 22, ou un anticorps selon la revendication 21.

26. Réactif de diagnostic éventuellement marqué, qui comprend un ADN isolé ou une sonde selon l'une quelconque des revendications 3 à 7, ou 9, une bactérie ou un virus selon la revendication 23, une protéine ou un peptide recombinant(e) ou synthétique selon la revendication 20 ou 22, ou un anticorps selon la revendication 21.

27. Réactif de diagnostic éventuellement marqué selon la revendication 26, immobilisé sur une phase solide.

28. Nécessaire pour essai de diagnostic comprenant, en tant que constituant, un réactif éventuellement marqué selon la revendication 26 ou 27.

29. Réactif de diagnostic selon la revendication 26 ou 27 ou nécessaire pour essai selon la revendication 28 pour le diagnostic de la maladie de Crohn chez les humains ou de la maladie de Johne chez les animaux.

## Revendications (Revendications pour l'(les) Etat(s) contractant(s) suivant(s): ES, GR)

1. Procédé d'obtention d'un élément d'insertion mycobactérien ou d'un fragment de celui-ci, qui comprend la détection de l'ADN d'une mycobactérie pathogène avec une sonde, ladite sonde comprenant :
(a) un ADN isolé essentiellement constitué d'une séquence d'ADN mycobactérien ayant au moins 70 % d'homologie de séquence avec un quelconque segment contigu d'au moins 20 nucléotides de le séquence d'insertion IS900 portée par pMB22 (NCIMB 12461) ;
(b) un ADN isolé ayant au moins 70 % d'homologie de séquence avec la séquence d'insertion IS900 entière portée par pMB22 (NCIMB 12461) ;
(c) un ADN isolé qui est la séquence d'insertion IS900 de pMB22 (NCIMB 12461) ;
(d) une sonde d'ARN ou d'oligonucléotide synthétique basé sue une séquence d'ADN de l'un quelconque de (a) à (c) ; ou
(e) une sonde comprenant un ADN selon l'un quelconque de (a) à (c) ou un ARN ou un oligonucléotide synthétique selon (d) portant un marqueur de détection;
pour identifier un élément d'une séquence d'insertion ou un fragment de celui-ci qui se sépare avec de la pathogénie mycobactérienne, et l'isolement dudit élément de la séquence d'insertion ou fragment de celui-ci.

2. Procédé pour détecter la présence ou l'absence d'une souche ou d'un phénotype mycobactérien, qui comprend l'essai d'un échantillon d'ADN ou d'ARN mycobactérien avec une sonde comprenant :
(a) un ADN isolé essentiellement constitué d'une séquence d'ADN mycobactérien ayant au moins 70 % d'homologie de séquence avec un quelconque segment contigu d'au moins 20 nucléotides de la séquence d'insertion IS900 portée par pMB22 (NCIMB 12461) ;
(b) un ADN isolé ayant au moins 70 % d'homologie de séquence avec la séquence d'insertion IS900 entière portée par pMB22 (NCIMB 12461) ;
(c) un ADN isolé qui est la séquence d'insertion IS900 de pMB22 (NCIMB 12461) ;
(d) une solide d'ARN ou d'oligonucléotide synthétique basé sur une séquence d'ADN de l'un quelconque de (a) à (c) ; ou
(e) une sonde comprenant un ADN selon l'un quelconque de (a) à (c) ou un ARN ou un oligonucléotide synthétique selon (d) portant un marqueur de détection ;
ou un élément d'insertion mycobactérien ou un fragment de celui-ci pouvant être obtenu par le procédé de la revendication 1.

3. Procédé selon la revendication 2 pour l'identification et la différentiation précises de souches et d'espèces mycobactériennes étroitement apparentées.

4. Procédé selon la revendication 3, dans lequel la souche ou l'une des souches mycobactériennes(s) est *M. paratuberculosis.*

5. Procédé selon la revendication 3, dans lequel la souche ou l'une des souches mycobactérienne(s) fait partie du groupe du complexe de *M. avium-intracellulare* ou de *M. avium.*

6. Procédé selon la revendication 3, dans lequel la souche ou l'une des souches mycobactérienne(s) est *M. leprae.*

7. Procédé selon la revendication 3, dans lequel la mycobactérie ou l'une des mycobactéries provoquent des surinfections associées au SIDA ou une tuberculose atypique.

8. Procédé selon la revendication 3, dans lequel le phénotype est la pathogénie ou la résistance aux médicaments.

9. Procédé selon la revendication 3, dans lequel la souche mycobactérienne provoque la maladie de Johne chez les animaux ou la maladie de Crohn chez l'homme.

10. Procédé selon l'une quelconque des revendications 3 à 9, dans lequel l'échantillon comprend des fluides corporels, un tissu, des extraits tissulaires, des excreta, des cultures *in vitro* ou des échantillons de l'environnement.

11. Procédé de détection de mycobactéries par une réaction en chaîne par polymérase, dans lequel on utilise un fragment pouvant être obtenu par le procédé de la revendication 1.
